# EUROPEAN PATENT APPLICATION

(11) **EP 2 604 600 A1**
(43) Date of publication of application: **19.06.2013**
(21) Application number: 11193462.6
(22) Date of filing: 14.12.2011
(51) Int. Cl.: C07D 401/12, C07D 403/12, A61K 31/403

(54) **Isatin derivatives and their use in therapy**

(71) Applicant: King Saud University, 11421 Riyadh (SA)
(72) Inventor: Mohamed Hassan, Tarek Aboul-Fadl, 11421 Riyadh (SA)
(74) Representative: Goddar, Heinz J.

(57) **Abstract**

The present invention relates to compounds of formula (I) 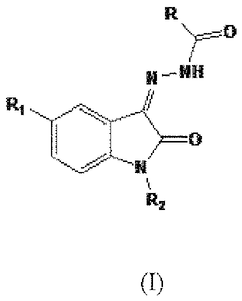 wherein R is pyridyl, R₁ is selected from Cl or NO₂ and R₂ is H; or R is 2-nitrofuranosyl, R₁ is selected from H, F, Cl, CH₃ or OCF₃ and R₂ is selected from H, phenyl or benzyl; associated pharmaceutical compositions and use of the compounds or compositions in therapy, in particular for the treatment of tuberculosis as well as a method for their preparation.

## Description

The present invention relates to isatin derivatives and their use in therapy.

Tuberculosis (TB) remains among the world's great public health challenges. Worldwide resurgence of TB is due to two major problems: the acquired immunodeficiency syndrome (AIDS) epidemic, which started in the 1980s, and the outbreak of multidrug-resistant (MDR)- and extensively drug-resistant (XDR)-TB. Thus, there is an urgent need for anti-TB drugs with improved properties.

In recent years, Schiffand Mannich bases of 1H-indoline-2,3-diones (isatines) were reported to exhibit anti-TB activity. Investigation of the structure-activity relationships in isatin derivatives revealed that 5-halogenation, N-alkylation and N-Mannich bases were effective in causing a marked enhancement in the activity against various bacteria. Furthermore, more recently a hypothetical pharmacophore model was built using Molecular Operating Environment (MOE) program. The built pharmacophoric model revealed the necessity of the following pharmacophoric features for the anti-TB activity: an aromatic center, hydrogen acceptor/metal ligator center, hydrogen bond donor center and a further aromatic center/hydrophobic area.

Aboul-Fadl et al. Eur. J. Med. Chem. 2010, 45, 4578-4586 relates to the synthesis of different Schiff bases of indoline-2,3-dione derivatives and their antitubercular activity.

Aboul-Fadl et al, Int. J. Res. Pharm. Sci. 2010,1,113-126 is a review article which is related to the anti-tubercular activity of isatin derivatives.

It is an object of the present invention to provide isatin derivatives having enhanced activity against MDR. strains, reduced toxicity, shortened duration of therapy, rapid mycobactericidal mechanism of action and/or the ability to penetrate host cells and exert antimycobacterial effects in the intracellular environment.

This object is achieved by a compound of formula (I) 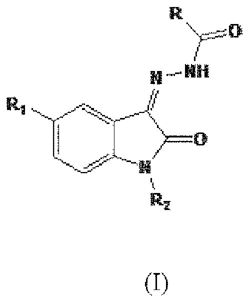 wherein R is pyridyl, R₁ is selected from Cl or NO₂ and R₂ is H; or R is 2-nitrofuranosyl, R₁ is selected from H, F, Cl, CH₃ or OCF₃ and R₂ is selected from H, phenyl or benzyl.

This object is also achieved by the inventive compound for use in therapy.

Further, the object is achieved by the inventive compound for use in the treatment of tuberculosis.

Moreover, the object is achieved by the inventive compound for use in the treatment of single resistant *mycobacterium tuberculosis.*

Furthermore, the object is achieved by a pharmaceutical composition comprising the inventive compound together with at least one pharmaceutically acceptable excipient.

Finally, the object is achieved by a method for preparing the inventive compound, wherein an isatin of the formula (II) is reacted with a carbohydroxide (III); and
R₁ is selected from Cl or NO₂ and R₂ is H in case that R is pyridyl; or
R₁ is selected from H, F, Cl, CH₃ or OCF₃ and R₂ is selected from H, phenyl or benzyl in case that R is 2-nitrofuranosyl.

Preferably, the reaction is carried out in the presence of an acidic catalyst, preferably in the presence of C₁-C₆ carboxylic acid, most preferably in the presence of acetic acid.

In a preferred embodiment, the reaction is carried out in an organic solvent, preferably an alcohol, most preferably ethanol.

Even preferred, the reaction is carried out under reflux conditions.

Finally preferred, the reaction is carried out under microwave irradiation.

It was surprisingly found that the inventive compounds solve the problem, particularly by exhibiting an enhanced activity against tuberculosis compared to the compounds of the prior art.

It was further surprisingly found that the inventive compounds can be prepared in an easy and efficient manner according to the inventive method.

The term "pharmaceutical composition", as used herein, is intended to comprise at least one of the inventive compounds. The pharmaceutical composition can be, for example, in a liquid form, e.g. a solution, syrup, elixir, emulsion and suspension, or in a solid form, e.g. a capsule, caplet, tablet, pill, powder, and suppository. Granules or semi-solid forms and gelcaps are also considered. In case that the pharmaceutical composition is a liquid or a powder, the dosage unit optionally is to be measured, e.g. in the dosage unit of a teaspoonfull. In addition to the inventive compound the pharmaceutical composition can comprise, for example, flavouring agents, sweeteners, dyes, preservatives, stabilizers, colouring agents, diluents, suspending agents, granulating agents, lubricants, binders and disintegrating agents. A tablet, for example, can be coated. A liquid to be injected should be sterile. All of the formulations mentioned can be intended for immediate release, time release and sustained release.

The term "pharmaceutically acceptable", as used herein, means at least non-toxic. The "pharmaceutically acceptable carrier", as meant in the present disclosure, may take a wide variety of forms depending upon the desired route of administration. The term comprises conventional pharmaceutical diluents such as water or ethanol and conventional tabletting ingredients such as corn starch, lactose, sucrose, sorbitol, talc, stearic acid, magnesium stearate, dicalcium phosphate or gums. Administration of the pharmaceutical composition of the present invention can use different routes such as oral, sublingual, parenteral, intravenous, intraperitoneal, nasal, vaginal, rectal, subcutaneous, intradermal, intramuscular and topic. A dosage unit can be administered once or several times a day, week or month. The delivery can also be continuously, for example by infusion or through a transdermal sustain release system.

The therapeutically active compounds should, preferably, be present in the above-mentioned pharmaceutical composition in a concentration of about 0.1 to 99.5, preferably of about 0.5 to 95% by weight.

The above-mentioned pharmaceutical formulations can also contain other pharmaceutical formulations; can also contain other pharmaceutical active compounds in addition to the active compound according to the invention.

The above-mentioned pharmaceutical formulation is prepared in the customary manner according to known methods, for example by mixing the active compound or compounds with the excipient or excipients.

Additional features and advantages of the present invention will become apparent in the following detailed description on the basis of examples.

### Example 1

### Synthesis of N-benzylisatin building blocks

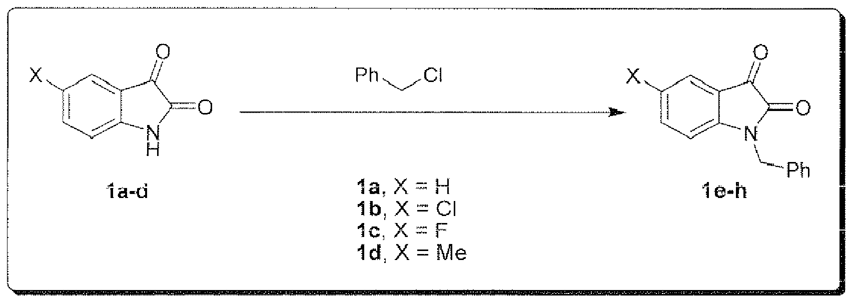

Other isatins were obtained commercially.

*Conventional method:* A flask equipped with a magnetic stirring bar was charged with 100 ml of DMF and potassium carbonate (1.8 g, 13 mmol). The mixture was stirred at room temperature for 5 minutes. Afterwards the appropriate isatin derivative 1a-d (10 mmole) was added. Stirring was continued for 45 minutes and then benzyl chloride (1.39 g, 11 mmol) was added. Stirring was continued at 80°C for 12 h and the mixture diluted with 200 ml of water. The mixture was extracted with three 100-ml portions of diethyl ether. The combined organic layers were washed with water (3x50ml), dried over calcium chloride, and the solvent was removed at slightly reduced pressure to yield 1-benzyl3satins 1e-h.

*Microwave Irradiation Method (MWI):* A mixture of isatin 1a-d (10 mmol), benzyl-chloride (1.39 g, I mmol), potassium carbonate (18 g, 13 mmol) and some drops of DMF was exposed to MW irradiation for 5 min at 200W (80°C). The reaction mixture was cooled to room temperature and mixed thoroughly with ice-water. The formed solid was purified as mentioned in the above method to give compounds 1e-h. Yields of both methods are given in Table 1,

**Table 1: Reaction times and yields for isatin N-alkylation under MWI and conventional heating.**

| Compound | conventional heating | | *MWI* | |
|---|---|---|---|---|
| | h/°C | Yield (%) | min/watts Yield (%) | |
| 1e | 12/80 | 70 | 5/200 | 95 |
| 1f | 12/80 | 65 | 5/200 | 90 |
| 1g | 12/80 | 73 | 5/200 | 96 |
| 1h | 12/80 | 56 | 5/200 | 80 |

### Example 2

### Synthesis of hydrazide building blocks

Isonicotinic acid hydrazide (2a) is commercially available.

### Synthesis of 5-nitrofuran-2-carbohydrazide (2b)

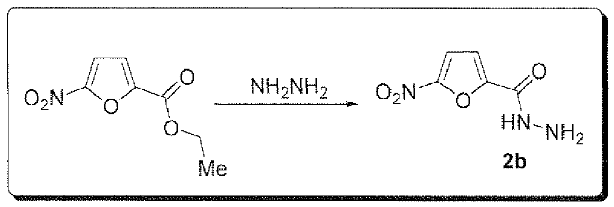

A solution of 99.9% hydrazine hydrate (0.55 g, 11 mmol) and ethyl 5-nitrofuran-2-carboxylate (1.85 g, 10 mmol) in absolute ethanol (100 ml) was stirred at 0°C for 1 h. Stirring was continued for further 1 h at room temperature. The formed solid was filtered off washed with ethanol and dried by suction. Crystallization from ethane) afforded the corresponding 5-nitrofuran-2-carbohydrazide (2b).

### Example 3

### General procedure for preparation of isatin derivatives

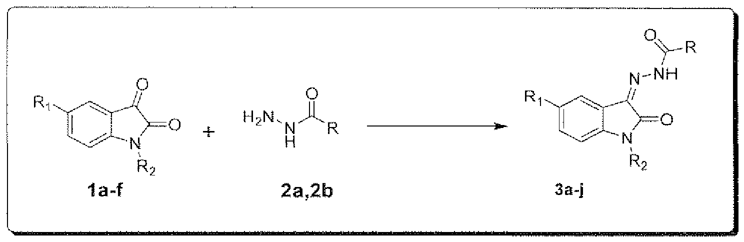

*Conventional method:* A mixture of appropriate acid hydrazide, 2a or 2b, (1 mmol) and appropriate isatin derivative, la-f, (1 mmol) in ethanol (25 mL) was refluxed in presence of glacial acetic acid (1 mL) for appropriate time (4-6 hours for complete reactions). The reaction mixture was cooled, the precipitated solid filtered, purified by flash chromatography and crystallized from appropriate solvent (ethanol or ethanol/DMF mixtures).

*kficrowave Irradiation Method (MWI)*: A solution of appropriate hydrazide 2a or 2b, (1 mmol) and appropriate isatin derivative, 1a-f (1 mmol) in ethanol (15 ml) was prepared. Few drops of glacial acetic acid were added and the whole reaction mixture was microwave irradiated at 90°C for 7 minutes. The reaction mixture was cooled and the resulting solid that separated on cooling was filtered, washed with cold ethanol, dried., purified by flash chromatography and recrystallized from the proper solvent.

Structures of the synthesized compounds were confirmed on the basis of spectral methods of analysis (IR, NMR and MS) .

### Example 4

### Evaluation of the anti-TB activity of the synthesized compounds

Anti-TB screening of all the synthesized Schiff bases, Resazurin MIC Assay with single resistant *MTB* and MBC assay with *MTB* H37Rv, was carried out at National Institutes of Health and the National Institute of Allergy and Infectious Diseases, Bethesda, MD 20892-6604, URSA. Contract No. HHSN266200600011C (N01-AI-60011).

### Resazurin MIC Assay with Sensitive and Single Resistant Mycobacterium tuberculosis and MBC Assay with_M. tuberculosis H37Rv of individual compounds

### Bacterial Strains

The following single resistant *M. tuberculosis* strains were obtained from the American. Type Culture Collection (ATCC), Manassas, VA:
ATCC 35837: ethambutol resistant
ATCC 35530: ethionamide resistant
ATCC 35822: isoniazid resistant
ATCC 35827: kanamycin resistant
ATCC 35821: para-aminosalicylic acid resistant
ATCC 35820: streptomycin resistant

The pan-sensitive *M. tuberculosis* H37Rv strain was obtained from Colorado State University, Fort Collins, CO.

### MIC Assay

The resazurin MIC assay, developed by Collins and Franzblau (1997), is a colorimetric assay used to test compounds for antimycobacterial activity. A color change from blue to pink is observed when growth occurs. Compounds are routinely tested at 8 dose levels in a dose range between 10 and 0.078 µg/ml. For special studies, MIC assays with single resistant bacteria are performed with compounds with known antimycobacterial activity. Therefore, the initial single concentration assay with a high dose of 10 µg/ml is not performed.

### Preparation of the inoculum:

The single resistant bacteria and H37Rv were grown in Middlebrook 7H9 broth medium (7H9 medium) supplemented with 0.2% (v/v) glycerol, 10% (v/v) ADC (albumin, dextrose, catalase), and 0.05% (v/v) Tween 80. The bacteria were inoculated in 50 ml of 7H9 medium in 1 liter roller bottles that were placed on a roller bottle apparatus in an ambient 37°C incubator. When the cells reached an OD600 of 0.150 (equivalent to ∼1.5 x 10⁷ CFU/ml), they were diluted 200-fold in 7H9 medium.

### Preparation of test compounds:

Stock solutions were prepared at 3.2 mg/ml which were then diluted as indicated below:
1. 20 µl of the 3.2 mg/ml stock of the test compound were added to a 96-well microtiter plate.
2. 2-fold dilutions were made by the addition of 20 µl of diluent.

| Expected final | Test Compound = 3.2 mg/ml | |
|---|---|---|
| dose level (µg/ml) | | Dilute 1:2 |
| 10 | 1^{st} dilution of 8 = 1.6 mg/ml | Dilute 1:2 |
| 5 | 2^{nd} dilution of 8 = 0.8 mg/ml | Dilute 1:2 |
| 2.5 | 3^{rd} dilution of 8 = 0.4 mg/ml | Dilute 1:2 |
| 1.25 | 4^{th} dilution of 8 = 0.2 mg/ml | Dilute 1:2 |
| 0.625 | 5^{th} dilution of 8 - 0.1 mg/ml | Dilute 1:2 |
| 0.312 | 6^{th} dilution of 8 = 0.05 mg/ml | Dilute 1:2 |
| 0.156 | 7^{th} dilution of 8 = 0.025 mg/ml | Dilute 1:2 |
| 0.078 | 8^{th} dilution of 8 = 0.0125 mg/ml | Dilute 1:2 |

3. Each dilution was further diluted 1:10 in sterile water (10 µl of dilution to 90 µl of sterile water). Note: The additional 10-fold dilution in water is required when DMSO is used as solvent to minimize toxicity to the bacteria. For uniformity in the assay procedure, this dilution step was used even if water or other solvents were used.

### Preparation of 96-well plates:

The following steps are followed in preparing the 96-wcll plates:
1. 6.25 µl of each dilution were transferred to duplicate 96-well test plates.
2. 93.75 µl of the cell suspension (∼ 10⁴ bacteria) in 7H9 medium were added to the test plates.
3. Positive, negative, sterility and resazurin controls were tested:
   a. Positive controls include: appropriate antibiotics for each resistant strain and rifampicin and isoniazid for 113 7Rv
   b. Negative controls include:
      i. cell culture with solvent and water
      i.i. cell culture only
   c. Sterility controls include:
      i. medium only
      ii. medium with solvent and water
   d. Resazurin control includes one plate containing the diluted compounds with resazurin only. No bacterial suspension is added. This control plate is needed to verify whether the compound reacts with resazurin that could possibly elicit fluorescence.
4. The 96 well test plates were incubated in an ambient 37°C incubator for 6 days.
5. After the 6 day incubation, 5 µl of a 0.05% sterile resazurin solution was added to each well of the 96-well plate. The plates are placed in an ambient 37°C incubator for 2 days.

### Evaluation of MIC values:

After the 2 day incubation, a visual evaluation for growth and color change in each well was performed. The MIC value was the lowest concentration of the test compound that visibly inhibited growth of the test organism. The results are expressed as µg/ml. Visible inspection of color change from blue to pink was also performed as a confirmation.

Compound 3b (see table 2) which is known from the prior art, was inlcuded in order to demonstrate the superiority of the inventive compounds.

Results of the compounds against pan-sensitive *M. tuberculosis* H37Rv strain are given in tables 2.

Results of the tested compounds against single drug resistant *M* . *tuberculosis* H37Rv strains are given in table 3.

### MBC Assay

The MBC assay with H37Rv was performed as an adjunct to the MIC assay and was used to determine the concentration of drug that is lethal to the target bacterium *in vitro.* A small aliquot (i.e., 10 µl) was removed from appropriate dilutions in the 96-well plate used to determine the MIC value with subsequent streaking on 7H10 agar plates. Five wells were selected, representing the well yielding the MIC value and 2 wells above and below the well that yielded the MIC value. After 3 weeks of incubation at 37°C, the agar plates were inspected for the presence or absence of growth. The MBC value is the concentration in µg/ml that inhibits ≥ 99.9% of the initial bacterial inoculum. Antimicrobials are usually regarded as bactericidal if the MBC is no more than four times the MIC. while the bacteriostatic agents have MIC values that are much lower than the MBC.

MBC results of the tested compounds are given in table 3.

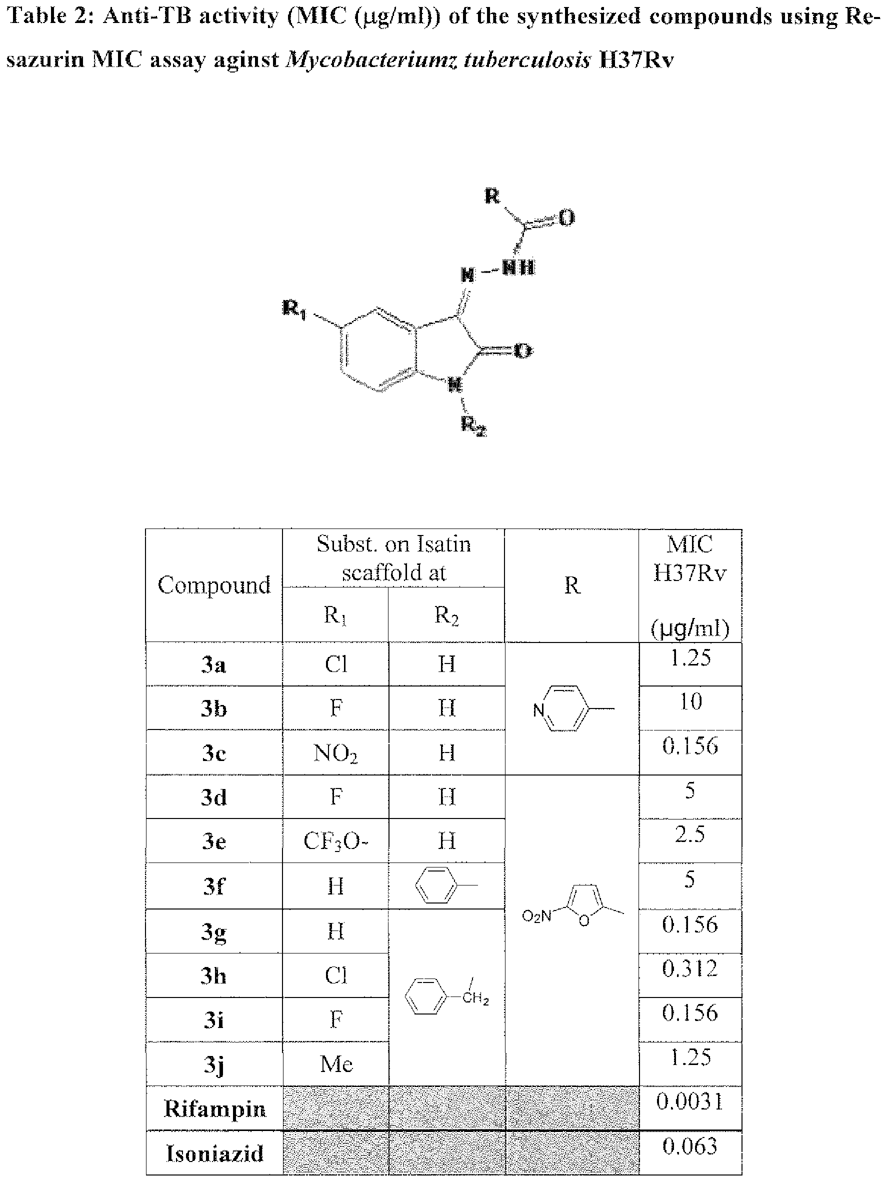

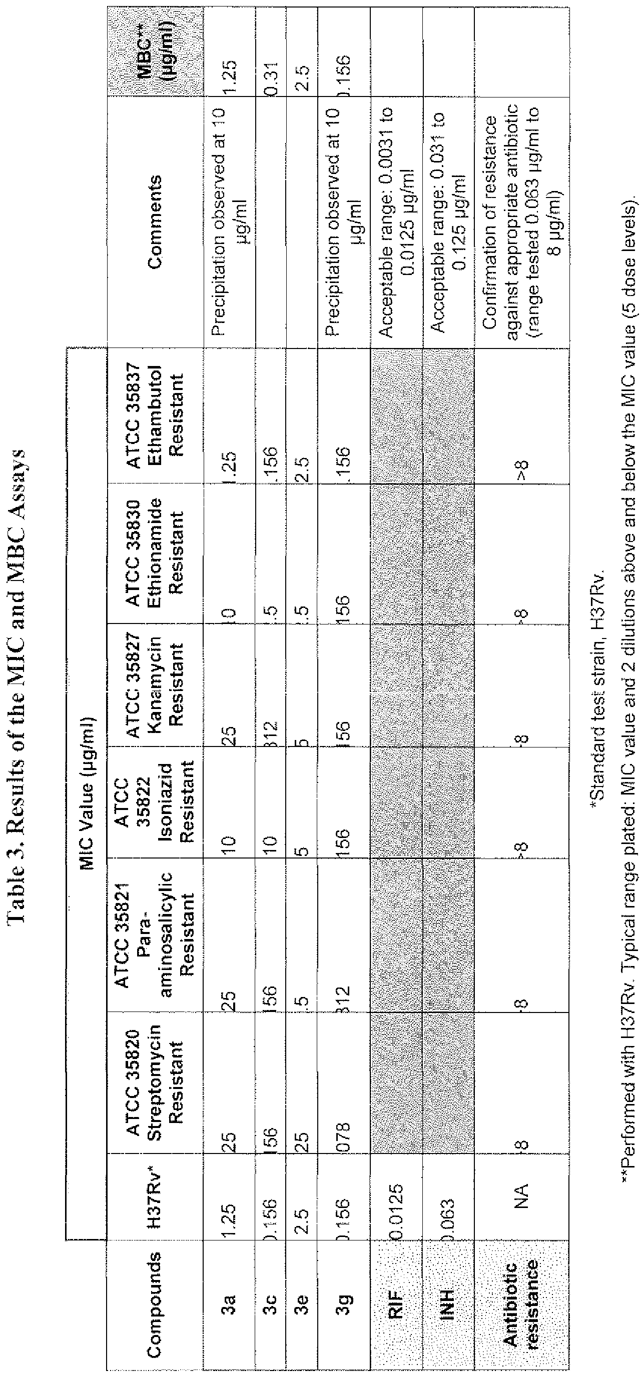

The features disclosed in the foregoing description and in the claims may both separately or in any combination be material for realizing the invention in diverse forms thereof.

## Claims

1. Compound of formula (I) 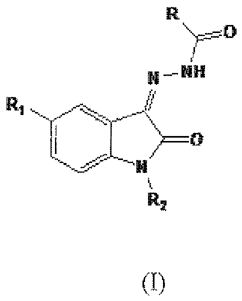 wherein R is pyridyl, R₁ is selected from Cl or NO₂ and R₂ is H; or
R is 2-nitrofuranosyl, R₁ is selected from H, F, Cl, CH₃ or OCF₃ and R₂ is selected from H, phenyl or benzyl.

2. Compound according to claim 1 for use in therapy.

3. Compound according to claim 1 for use in the treatment of tuberculosis.

4. Compound according to claim 1 for use in the treatment of single resistant *mycobacterium tuberculosis.*

5. Pharmaceutical composition comprising a compound according to claim 1 together with at least one pharmaceutically acceptable excipient.

6. Method for preparing a compound according to claim 1, wherein
an isatin of the formula (II) is reacted with a carbohydroxide (III); and 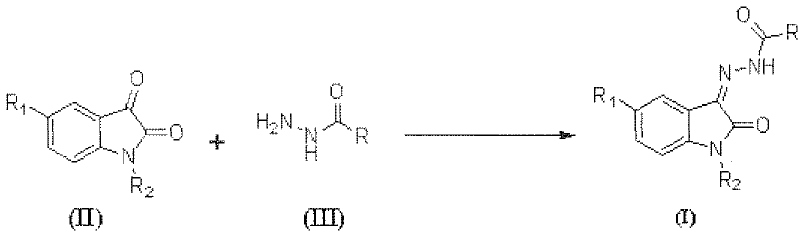 R₁ is selected from Cl or NO₂ and R₂ is H in case that R is pyridyl: or
R₁ is selected from H, F, Cl, CH₃ or OCF₃ and R₂ is selected from H, phenyl or benzyl in case that R is 2-nitro furanosyl.

7. Method according to claim 6, wherein the reaction is carried out in the presence of an acidic catalyst, preferably in the presence of C₁-C₆ carboxylic acid, most preferably in the presence of acetic acid.

8. Method according to claim 6 or 7, wherein the reaction is carried out in an organic solvent, preferably an alcohol, most preferably ethanol.

9. Method according to any of the claims 6 to 8, wherein the reaction is carried out under reflux conditions.

10. Method according to any of the claims 6 to 8, wherein the reaction is carried out under microwave irradiation.
